(19) Europäisches Patentamt
European Patent Office
Office européen des brevets

(11) **EP 3 629 883 B1**

(12) **EUROPEAN PATENT SPECIFICATION**

(45) Date of publication and mention
of the grant of the patent:
**28.10.2020 Bulletin 2020/44**

(51) Int Cl.:
***A61B 3/00*** *(2006.01)*       ***A61B 3/18*** *(2006.01)*
***A61B 3/032*** *(2006.01)*

(21) Application number: **18733342.2**

(22) Date of filing: **28.05.2018**

(86) International application number:
**PCT/IB2018/053777**

(87) International publication number:
**WO 2018/220508 (06.12.2018 Gazette 2018/49)**

(54) **DEVICE AND METHOD FOR THE EVALUATION OF VISUAL ABILITIES**

VORRICHTUNG UND VERFAHREN ZUR BEURTEILUNG VON SEHFÄHIGKEITEN

DISPOSITIF ET PROCÉDÉ POUR L'ÉVALUATION DE CAPACITÉS VISUELLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.05.2017 IT 201700058612
29.11.2017 IT 201700137065**

(43) Date of publication of application:
**08.04.2020 Bulletin 2020/15**

(73) Proprietor: **Zingara Limited
London SE1 2SX (GB)**

(72) Inventor: **SANGIUOLO, Raffaele
84134 Salerno (IT)**

(74) Representative: **Papa, Elisabetta et al
Società Italiana Brevetti S.p.A
Piazza di Pietra, 39
00186 Roma (IT)**

(56) References cited:
**EP-A1- 2 669 732       US-A1- 2008 189 173
US-A1- 2010 168 606       US-A1- 2011 027 766
US-A1- 2012 075 586       US-A1- 2016 220 162**

**Description**

**Technical field of the invention**

The present invention relates to a viewer device and a method, based on digital technology, for the evaluation of the visual abilities of a subject.

**Background**

The evaluation of a person's visual abilities is fundamental in many areas, both private, for example for the achievement of a driving license, and professional, in particular for the suitability to specific types of tasks, and also for sports, where high visual performance is required.

Despite the social and professional impact of the visual ability of the subject, to date the different parameters that contribute to define in a complete and exhaustive manner such ability - such as mono- and bi-ocular visual acuity, sensitivity to contrast and more - are measured and evaluated separately, often in an uncoordinated manner and in some details in a superficial manner.

In particular, to the Inventors' knowledge, a digital instrument suitable for allowing the execution of a multifactorial test that takes into account, in a unique and synergic way, the different factors that define the visual ability of the subject, possibly also in relation to a specific activity, is not available.

Likewise, to the Inventors' knowledge, a globally representative index of said different factors contributing to this visual ability has not been defined.

It should also be noted that also the measuring tests and instruments currently available for the evaluation of a single visual parameter often provide inaccurate and non-repeatable data, as they are influenced by the environmental conditions of the test.

US 2011/027766 discloses a kiosk with a display for far vision tests rigidly hold and a sliding display for near vision tests.

US 2008/189173 discloses a kiosk with two movable displays for far and near vision tests.

**Summary of the invention**

The technical problem posed and solved by the present invention is therefore that of providing a device and a method for assessing the visual ability of a subject which allow overcoming the drawbacks mentioned above with reference to the prior art.

This problem is solved by a viewer device according to claim 1.

Preferred features of the present invention are the subject of the dependent claims.

The inventors have devised a device and a (non-claimed) method that allow, jointly or independently of each other, the evaluation of the visual ability of a subject in an objective, accurate and repeatable manner, also possibly with reference to a specific activity. This is achieved by measuring a plurality of factors, or parameters, that contribute to determining the overall visual capacity.

The invention provides a viewer device, in particular based on digital technology, for the evaluation of a plurality of factors expressing the visual ability of the subject and preferably representative of the aspects that contribute to the dynamic vision during ordinary and extraordinary daily activities.

The viewer device of the invention has some significant advantages.

First of all, the device allows, in a single apparatus, the evaluation of several factors that contribute to the visual ability. This makes the exams faster, simpler, less expensive and that can also be performed by non-specialized operators. Moreover, it allows evaluating said parameters, in an accurate and repeatable manner, without the environmental artifacts that afflict already known measurement systems.

Furthermore, the device lends itself to being made in a configuration of minimum weight and bulk, which is transportable and easy to assemble on site.

In a particularly preferred implementation, the method and the device of the invention allow the calculation of a multifactorial visual index, which can also be defined global and/or dynamic, suitable for quantifying the visual ability of a subject by combining different parameters, each representative of a specific capacity. According to an embodiment, the value of this index is automatically (expressed numerically). This numerical expression can also be, for example to exemplify enabling procedures, entered at the end of the examination on a predefined scale of values, in particular made up of five different levels of visual quality. The definition of the quality level related to the vision of the examined subject within the evaluation scale allows facilitating enabling criteria which can be differentiated according to specific needs, for example by setting "ad hoc" thresholds.

In a particularly advantageous embodiment, the device and the method of the invention, possibly in relation to the aforementioned global visual index, are used for the evaluation of the visual ability of drivers of road vehicles and/or of agonistic drivers of motor vehicles, cars or other motor vehicles, including boats.

The device and the method of the invention allow calculating also a visual skill index other than the one considered above and/or dedicated to different activities, thus modulating the values of relative importance of each visual factor that contributes to them. For example, an index can be developed to assign relative enabling levels in the context of professional activities that require specific skills.

In the latter application, the visual index calculated by the device and the method of the invention allows improving the current enabling methods, often based on incomplete tests that do not detect visual factors that come into play very significantly during activities of interest. In the specific case of the guide, such factors are, for example, in addition to the measurement of visual acuity, quality of the visual field, stereopsis, contrast sensitivity and dazzling recovery time. Moreover, some national statistics indicate that many traffic accidents are caused by problems related to the driver's vision.

Further specific applications of the device and of the method of the invention, possibly in association with the aforesaid visual index, may, for example, relate to other competitive sports. In this case, the invention can advantageously allow a better selection of the athletes.

Another field of application of the invention, and in particular of the proposed visual index, is that of legal medicine and insurance, since the visual ability can contribute to the dynamics of an accident and therefore influence the criteria of responsibility and indemnity.

Likewise, in the field of occupational medicine, the evaluation of visual abilities, and especially of the visual index as defined above, allows a better selection of personnel, reporting the visual qualities to the type of activity they are required to perform at the time of recruitment.

Further advantages, features and methods of use of the present invention will be apparent from the following detailed description of some embodiments thereof, made by way of a non-limiting example.

## Brief description of the figures

Reference will be made to the figures of the accompanying drawings, in which:

- Figure 1 shows a side view of a preferred embodiment of a viewer device according to the present invention;

- Figure 2 shows a top plan view of the viewer device in Figure 1;

- Figure 3 shows a front view of the viewer device in Figure 1;

- Figures 4A and 4B show each a schematic cross-sectional view of the viewer device in Figure 1, in a respective operative configuration;

- Figure 5 exemplifies, by means of a block diagram, a preferred embodiment of processing means of the viewer device in Figure 1 or of that in the subsequent Figures 8A-8C;

- Figure 6 shows a schematic representation of a mode of implementation of a visual field test that can be performed with the viewer device in Figure 1 or that in the subsequent Figures 8A-8C;

- Figure 7 shows an exemplary flow chart of methods for automatically calculating, in digital, a visual index, in particular obtainable with the viewer device in Figure 1 or with that in the subsequent Figures 8A-8C; and

- Figures 8A, 8B and 8C relate to another preferred embodiment of a viewer device according to the present invention, showing a front perspective view, a side perspective view and a rear perspective view, respectively;

- Figure 9 shows a flow chart illustrating a preferred embodiment of an algorithm for calculating a Global Visual Index obtained by means of the viewer device in Figure 1 or of that in Figures 8A-8C.

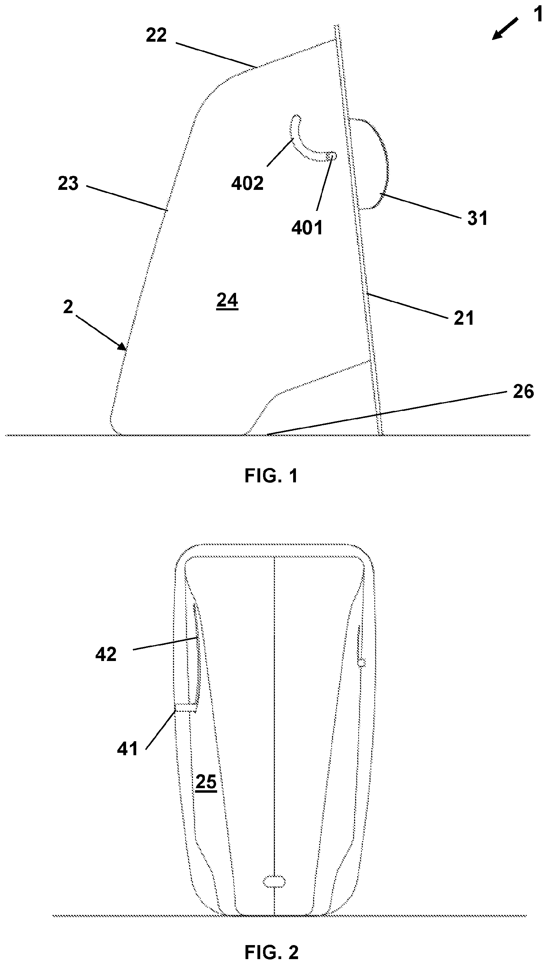

The dimensions and curves shown in Figures 1 to 4B and 8A o 8C above are to be understood as examples and are not necessarily shown in proportion.

## Detailed description of preferred embodiments

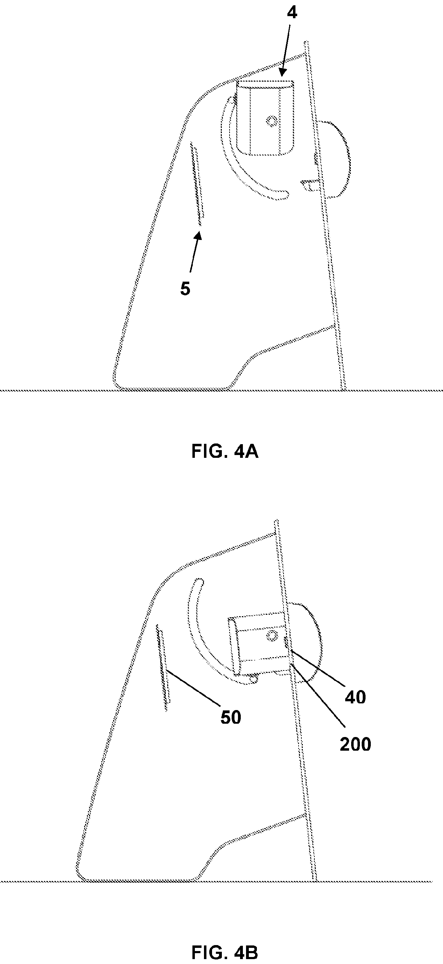

With reference initially to Figures 1 to 4B, a viewer device according to a preferred embodiment of the invention is denoted as a whole with reference numeral 1. The viewer device 1 is configured to allow the execution of a multifactorial visual ability test on a subject.

The viewer device 1 comprises an outer casing 2, which in the present example is shaped in the form of an irregular polyhedron defined by a plurality of walls, or surfaces, preferably connected to one another by means of curved profiles.

In particular, the casing 1 has a front wall 21, an upper wall 22 connected with a rear wall 23, a first and a second side wall, respectively denoted by 24 and 25, and a lower wall 26.

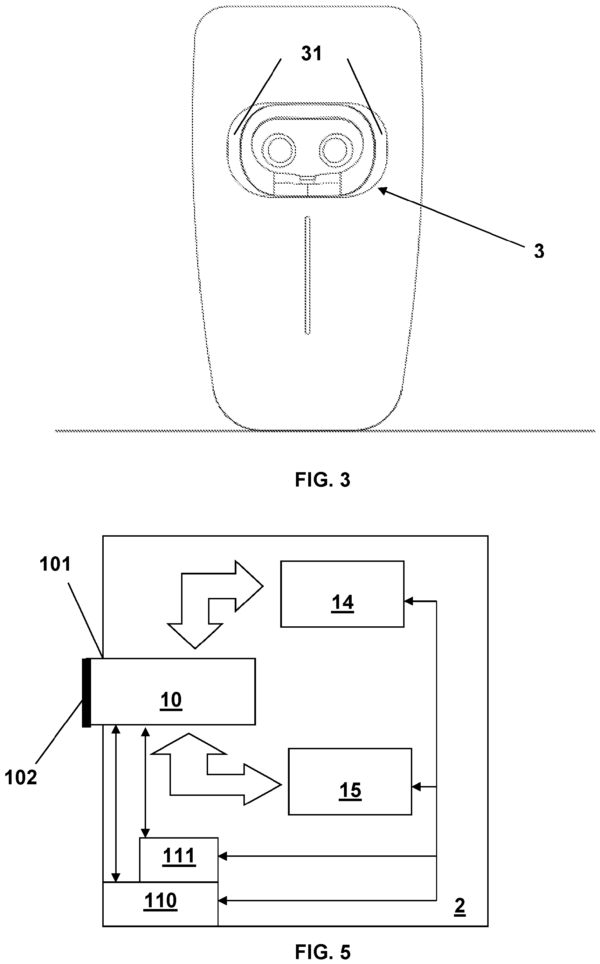

On the outer casing 2, at the front wall 21, a visual access window 3 is formed so as to house at least the upper part of the head of the subject and is better visible in Figure 3. Preferably, the visual access window 3 is screened by side walls 31 and may be associated with a chin support.

The opening of the window 3 can be screened by means of a lens or a different transparent panel.

Preferably, between the window 3, and therefore the eyes of the subject examined, and the display 40, a seat 200 is obtained for the application of any corrective lenses, for example to compensate for visual defects of the patient. The latter can easily be interchanged manually, for example by locking clips.

At the window 3, inside the casing 2, the viewer device 1 comprises first display means 4, in particular having a first display preferably at high resolution. The display can be mono-ocular or bi-ocular, in this last case possibly with a physical or digital partition between the images to be administered to the two eyes.

The first display means 4 preferably comprise focusing means, for example biconvex lenses, optionally adjustable from the outside, for example by means of a pin 401 movable within a guide groove 402 formed on the side wall 24 of the casing 2.

The first display means 4 are movable between a first rest configuration, shown in Figure 4A, in which they are spaced from the window 3 and not interfering with the subject's field of view, and a second operative configuration, shown in Figure 4B, in which they are arranged in the subject's field of view and in front of the latter's eyes at the window 3.

In the present embodiment, the movement of the display means 4 from the first to the second configuration is obtained mechanically, by coupling a pin 41 integral with a main body of the means 4 with a guide 42 formed on the side wall 25 of the casing 2.

The viewer device 1 then comprises second display means 5, in particular including a second display 50 arranged in the proximity of the rear wall 23 within the casing 2.

The display 50 is, in use, in a more distanced position from the window 3 and from the patient's eyes with respect to the first display 40 and is therefore suitable for the evaluation visual abilities "from afar".

In a variant embodiment, an inter-pupillary distance adjustment system is provided. This system can be mechanically driven, for example of the pin 401 - guide 402 type mentioned above.

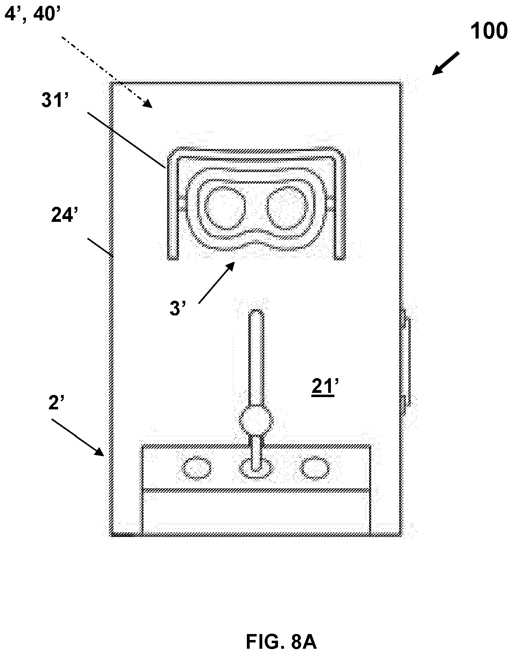

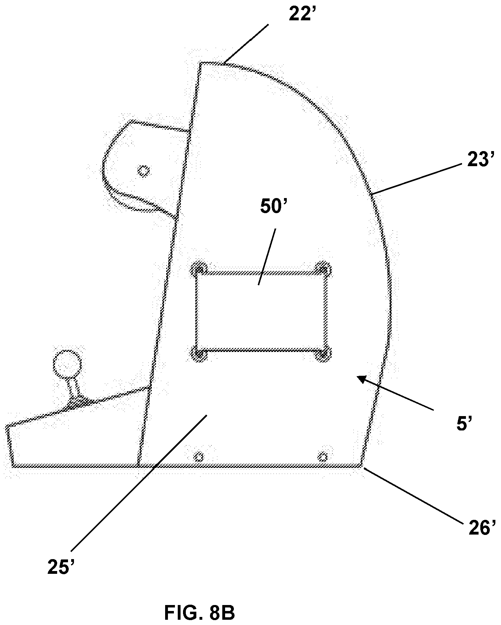

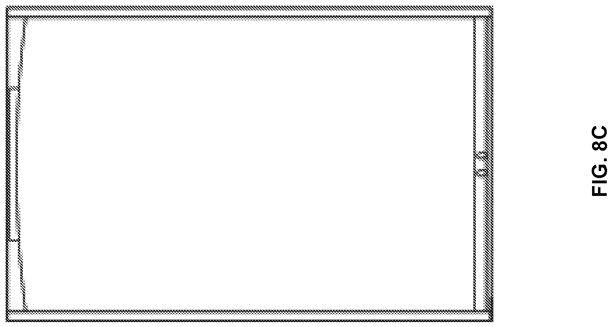

Figures 8A to 8C show another preferred embodiment of the viewer device according to the invention, which in this case is denoted as a whole with reference numeral 100. This viewing device 100 will be described below only with reference to the differences with respect to the first embodiment. In particular, in the above figures, components similar to those of the device 1 are denoted with the same numeral reference already used, with the addition of the apex " ' ". As mentioned, unless otherwise specified below, the same description given above applies to these components. In particular, the device 100 comprises a casing 2', a visual access window 3', a seat and related component or associated elements similar to those already described.

The device 100 comprises first display means 4' which differ from the means 4 described above in that they can be integral with the casing 2' and not movable with respect thereto. The first display means 4' of the device 100 can therefore assume a single operative configuration, in which they are arranged in the field of view of the subject and in front of the latter's eyes at the window 3'.

Therefore, in the device 100, means for moving the first display means 4' may correspondingly be absent.

The viewer device 100 then comprises second display means 5', in particular including a second display 50', which in the present example is arranged at or in the proximity of the side wall 24' or 25' of the casing 2', on the outer face of said wall. In use, the patient, or in general the subject to be evaluated, can be positioned so that the display 50' is in a more distanced position from him/her with respect to the first display means as accessible through the window 3'. Therefore, also this second display 50' is suitable for evaluations of visual capacities "from afar" and can be for this purpose programmed and/or controlled by a processing unit which will be described hereinafter.

The second display 50', advantageously, can also be used by an operator to impart commands to the device 100 and/or only to the second display means 5' or to control the examination procedure, and in this sense it can also implement a panel control, also introduced hereinafter.

Also in this embodiment an inter-pupillary distance adjustment system of the type already described may be provided.

In a variant embodiment, it is possible to perform a visual acuity test from a distance, or possibly a different test, by means of the first display means 4', in particular the relative display, appropriately controlled or programmed for this purpose.

As exemplified in the block diagram in Figure 5, the viewer device 1, 100 may also have a processing unit, for example comprising one or more (micro)processors, associated with the first and/or second display means 4, 4' and 5, 5' to administer one or more predetermined visual tests to the patient. Figure 5 shows, by way of example, processing means 14 and 15 as associated with the first and second display means 4, 4' and 5, 5', respectively.

In a preferred embodiment, the processing unit of the viewer device 1 also comprises a central control unit 10, preferably in communication with the processing means 14 and/or 15, where provided.

The control unit 10 can be managed directly by an operator, who works locally or remotely by means of an appropriate communication line.

A control panel 102 can be associated to the control unit 10, for example in the form of a touch screen and/or keyboard, in particular for receiving the inputs of an operator.

The control unit 10 can be placed inside the casing 2, 2' or at a further window thereof, for example a window 101 obtained at a side wall and exemplified in Figure 5.

Alternatively, the control unit 10 can be arranged in a remote position with respect to the casing 2, 2' and be considered part of the device 1, 100 itself or of an apparatus that includes it.

Preferably, the control unit 10 and/or the means 14 and 15 have processing and memory capacities capable of handling diagnostic images/videos proposed to the subject through the display means 4 and 5.

In general terms, the processing unit can be managed by an operator to present diagnostic tests to the subject and record the results.

As mentioned above, advantageously the device 1, 100 includes one or more memory units 110, preferably associated with the processing unit.

As already mentioned, the control unit 10 and the processing means 14 and 15 can be incorporated into a single processing unit, possibly including sub-units placed at the level of different components of the device 1, 100 itself and/or of an apparatus that includes it.

Advantageously, the viewer device 1, 100 provides, in association with the control unit 10 and/or the display means 4, 4' and 5, 5' introduced above or independently, connectivity means, for example Wi-Fi and/or LAN. A USB port, or equivalent, may also be provided for data export.

This connectivity can be used to communicate with any complementary accessories and/or with a server dedicated to assistance, updates, archiving, data processing, connection with other similar devices for data sharing or other purposes.

Embodiment variants may provide one or more tactile feedback devices 111, for example buttons and/or joysticks that the subject can use during the test to interact with the operator, the control unit, the display means and/or, in general, the processing unit.

In the present embodiment, the device 1, 100 is configured to administer tests related to the evaluation of one or more of the following factors to the patient:

- natural and corrected monocular visual acuity of both eyes,

- natural and corrected binocular visual acuity,

- visual field quality, both monocular and binocular,

- degree of binocular vision and qualitative evaluation of stereoscopy,

- heterophoria degree,

- contrast sensitivity,

- dazzling recovery time,

- chromatic sense quality,

- ocular motility through the Hess screen.

According to preferred operating modes, the device 1, 100, or the apparatus that includes it, are used in the context of the evaluation of the visual ability of the subject which provides the following preliminary steps:

- entering to a memory unit of, or associated with, the device 1, 100 personal data of the subject to be examined, also in order to determine the age for optimizing the tests;

- possible adjustment of the inter-pupillary distance to adapt it to that of the subject, in order to optimize the vision of the images presented,

- possible application of corrective lenses, if the subject has refractive defects, which are placed in a special support placed between the patient's eyes and the first or second display means 4, 4' and 5, 5', as mentioned above.

Preferred modes of execution of some of the tests cited above are illustrated below, purely by way of example and with particular reference to a method of evaluating the visual ability of the subject examined.

## Monocular and binocular visual acuity test

Preliminarily, the monocular visual acuity test is performed because if a visual acuity is found in the eye worse than or less than 2/10, a handicap will automatically be applied to the value of the global visual index calculated by the viewer device equal to 10% of its value.

Three types of optotypes can be adopted, such as letters, Albini's Random Dot E and numbers presented either in monocular vision or binocular simulated vision or performed from a distance, thus via the second display means 5, 5'. Each of the three tests consists of ten rows of symbols that have a decreasing size according to a logarithmic progression.

A line is considered perceived when the examined subject perceives at least 3 symbols out of 5 of it. The result is expressed through a numerical value ranging from 1 to 10 based on the line of symbols of smaller size perceived by the patient. This numerical value will be automatically expressed in an evaluation chart either individually or incorporated into the production calculation of a Global Visual Index which will be described later.

The values obtained from the examination of this parameter are expressed numerically. The numerical value is linked to the level of the smaller size of the symbols that the subject is able to perceive. For example, if a subject perceives at most the fourth row of the optotype adopted for the measurement of visual acuity, the numerical value that expresses it will be 4. The base value for the calculation of its Global Visual Index will be linked exclusively to this value. The evaluation of this result can also be entered by way of example, for example in the case of vehicle driving qualification procedures, at the end of the examination on a predefined scale of values, in particular made up of seven different levels at decreasing quality, as indicated in Table 1 below:

| Table 1 | |
|---|---|
| Level | Value |
| Band 1 | 10/10 |
| Band 2 | 9/10 |
| Band 3 | 8/10 |
| Band 4 | 7/10 |
| Band 5 | 6-5/10 |
| Band 6 | 4-3/10 |
| Band 7 | <3/10 |

As mentioned above, for the evaluation of the visual acuity from a distance, the second display means 5, 5' are adopted, excluding, in the case of the viewer device 1, the first means 4 by the mechanical system described above. As mentioned above, in the case of the viewer device 100 for the evaluation of visual acuity from a distance, the same external display normally used by the examined subject is used to control the exam procedure (for example 7 or 10 inches in size) or, in an alternative embodiment, the first display means 4' are used, in particular the relative display.

Then, the evaluation test of visual acuity in monocular vision is performed, sequentially excluding each of the two eyes, with identical methods to those adopted for the examination of the binocular visual acuity. The result related to this test may not be reported in the constitution of the aforementioned Global Visual Index.

## Visual field test

The examination of the binocular visual field is preferably carried out taking into consideration 77 points located in an area of 105° in the horizontal plane, 38° in the upper quadrants and 48° in the lower ones.

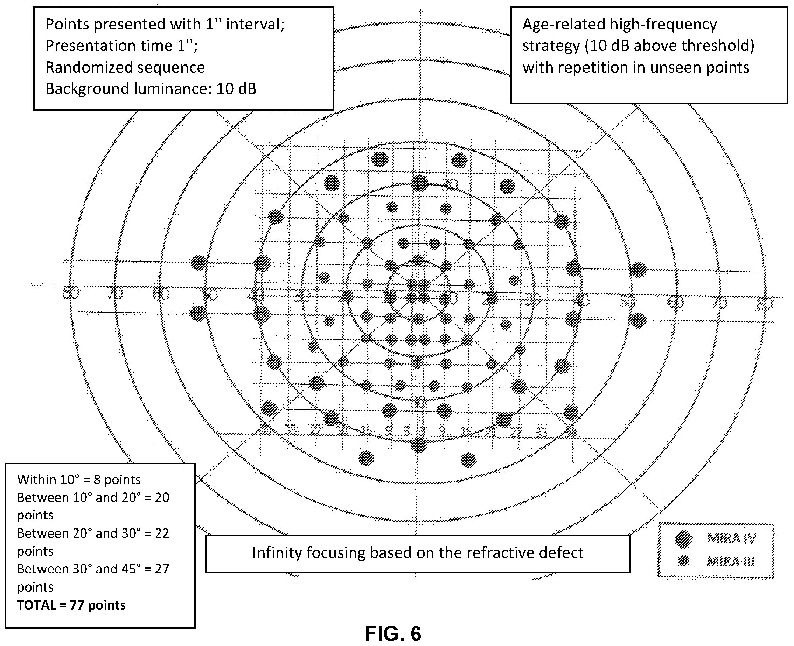

The sights, which are presented in a randomized sequence, have a decreasing luminosity from the periphery towards the center. A diagram related to the execution of this test is presented in Figure 6. The latter also indicates an exemplary mode for weighing the test result.

The presentation time is preferably about 0.3 s.

The response time, intended as patient feedback, is preferably about 1 s.

The interval between two successive presentations is preferably random and preferably included in a range of about 0.2-1 s.

The presentation sequence is preferably random.

Undetected points are re-presented a second time at the end of the cycle.

As for the results, the percentage coefficient of the points perceived compared to the total of those presented (respecting the weighing of the points) allows defining the numerical value related to this factor that is included in the calculation of the Global Visual Index at the end of the examination. It may also be entered by way of example, for example in the case of vehicle driving qualification procedures, at the end of the examination of values in one of the seven exemplary quality scale assessment bands according to the methods of Table 2 below.

The device 1, 100, or the apparatus that includes it, can show an operator:

- the percentage coefficient of the points detected compared to the total of those presented,

- the count of false positives,

- the count of the points seen,

- the graphical representation of unseen points,

- undetected points at a first examination are presented a second time at the end of the first cycle.

| Table 2 | |
|---|---|
| **Level** | **Coefficient** |
| Level 1 | 100% |
| Level 2 | 99-90% |
| Level 3 | 89-75% |
| Level 4 | 74-58% |
| Level 5 | 57-42% |
| Level 6 | 41-25% |
| Level 7 | <25% |

Three types of examination may also be provided according to the various age groups (for example: 0/40, 41/60 and over 60 years), selected by the device 1, 100 or by the related apparatus, which automatically calculates them from the master set. In this way, the physiological variation of the detection threshold that decreases with age is taken into account.

The contrast of the sights presented with respect to the background, which may for example exhibit a luminance of 1 Lux (31.5 ASB), will preferably increase according to Table 3 below.

| Table 3 | | | | |
|---|---|---|---|---|
| **Age range** | **Points up to 10** | **Points 10 to 20** | **Points 20 to 30** | **Points 30 to 40** |
| 0-40 | 8% | 16% | 32% | 48% |
| 41-60 | 10% | 20% | 40% | 54% |
| Over 60 | 12% | 24% | 48% | 60% |

To ensure that the subject examined correctly stares at the central point, at the same time a series of symbols can be presented in succession and randomly, the variation of which must be referred to the examined subject from time to time.

The percentage value is entered by the operator in the aforementioned memory unit, which can automatically place it in one of the bands shown above.

The visual field can also be examined in monocular vision by excluding each of the two eyes in succession. The grids used for this examination have the same characteristics as those used for binocular examination.

The important innovation consists in having adopted for the first time the evaluation criterion through a viewer device, preferably with digital technology, of the useful binocular visual field.

Evaluation of any heterophoria

This examination is carried out through the evaluation of the location of the perception by the examined subject of an aim with respect to a numbered bar for horizontal heterophores and to notes of a pentagram for vertical ones. The aim can be presented to one eye and the numbered horizontal scale or the pentagram to the contralateral one. If no deviation exists, the aim will fall at the center of them. The magnitude of a possible horizontal heterophoria is calculated by evaluating the magnitude of the deviation of the aim with respect to a horizontal scale with values between -10 (for exophores) and +10 (for esophores). The evaluation of any vertical heterophoria takes place by measuring the magnitude of the displacement of the aim with respect to a pentagram of vertical notes with values between -3 diopters for hypofory and +3 dioptres for hyperforie.

The value of these possible deviations is entered into the aforesaid memory unit, which extrapolates it for the evaluation of this single factor. The latter, in the present example, is not included in the Global Visual Index.

Stereoscopy examination

Its presence can be tested by presenting to the two eyes the image of two cubes of which one of different color in one eye with respect to the other and another of a third cube of the same color for both centrally located. In the case of presence of stereoscopic vision, three cubes will be perceived for the superimposition of the two isochromatic central units while the upper one and the lower one of different color will be perceived separately. If the stereoscopic vision is absent, on the other hand, 4 cubes will be perceived due to the non-overlap of those of the same color.

Since stereoscopic perception is the maximum expression of binocularity, it also requires quantitative evaluation. For the first time, this is done through a viewer using the "random-dot" test which is currently the most accurate evaluation system. All this has been possible thanks to digital technology that allows a much greater definition of the images compared to the evaluation systems adopted so far. This test is performed by creating a series of stereograms induced by moving horizontally two identical patterns presented simultaneously to the two eyes. This shift induces, if stereoscopy is present, a sensation of relief as groups relating to the image perceived by one eye merge with the corresponding ones perceived by the other eye. The lower the degree of perceived displacement that induces a sensation of relief the greater the degree of stereoscopy present. In order to perform this test, in the present example, 5 images induced to difficulties of increasing stereoscopic perception are foreseen. The most strereoscopically complex perceived image defines the degree of stereoscopy present. It is measured by the horizontal displacement evaluation expressed in seconds.

For this type of evaluation it is possible to foresee 5 different levels evaluated through the presentation of 5 different images presented in succession in a randomized way and with difficulty of decreasing stereoscopic perception, according to the following Table 4.

| Table 4 | |
|---|---|
| **Quality level** | **Entity of displacement** |
| Level 1 | 20" |
| Level 2 | 40" |
| Level 3 | 80" |
| Level 4 | 160" |
| Level 5 | 320" |

The level of stereoscopy detected can be viewed by the operator and entered into the aforementioned Global Visual Index. The evaluation coefficient of this factor used for the definition of the Global Visual Index is calculated by linking it to the level in the scale described above.

Evaluation of contrast sensitivity

This factor is preferably measured by presenting five quintets of symbols in a sequence (Albini's E of the same size as the fourth row of the optotype used to measure visual acuity) with a contrast decreasing with respect to the background and therefore increasingly difficult to perceive. The series of less contrasted images perceived (at least 3 symbols out of 5), within a time of 10" from the presentation - preferably calculated automatically by the viewer device or by an apparatus that includes it - defines the degree of sensitivity present, according to the following Table 5.

| Table 5 | |
|---|---|
| **Quality level** | **Perceived contrast** |
| Level 1 | 0.5% |
| Level 2 | 2% |
| Level 3 | 4% |
| Level 4 | 8% |
| Level 5 | 16% |

Also in this case, the operator can enter in the memory unit the value relating to the minimum degree of perceived contrast (at least three of the five symbols presented). Also in this case, the evaluation coefficient used for the definition of the Global Visual Index is calculated by linking it to the level in the scale described above.

Evaluation of dazzling recovery time

It is preferably calculated by making the examined subject get used to full dark for one minute and then dazzling him/her preferably for 30 s with a diffused light, preferably with a light intensity of 100 lux. During the dazzling step, symbols are presented at the center of the illuminated display that the subject will have to report to the examined subject so that he is forced to fix constantly in the center of the dazzling area, avoiding the possibility of fixation displacements that would modify the degree of induced dazzling. Then, the series of 5 symbols of the dimension equal to the fourth row of the optotype is presented, with a contrast equal to the minimum one perceived by the same subject in the previous test related to the evaluation of the contrast sensitivity. From the moment in which the induced dazzling ceases, the time taken to read at least 3 out of 5 of the symbols presented is measured.

The dazzling recovery quality is evaluated through the time taken to perceive the images expressed in seconds and its evaluation is divided into 3 different levels, as exemplified in Table 6 below.

| Table 6 | |
|---|---|
| Level 1 | recovery time < 20 s |
| Level 2 | recovery time 21 to 35 s |
| Level 3 | recovery time more than 35 s |

The device 1, 100 or the apparatus that includes it can provide to calculate the relative quality level of the dazzling recovery from the number of seconds needed to perceive the symbols whose value the examined subject will enter into the system itself. Also in this case, the evaluation coefficient used for the definition of the Global Visual Index is calculated by linking it to the level in the scale described above.

The great advantage, both at the level of the contrast sensitivity analysis and of the dazzling recovery time performed through the viewer device 1, 100 compared to the classical evaluation systems is that linked to the possibility of being able to perform both these tests modulating with extreme precision all the parameters related to the examination. In particular, it is possible to create a situation of absolute darkness in a first step and quantify in a very precise manner the amount of dazzling induced in a second step.

This is absolutely not possible with other systems because in the open environment, where these tests are commonly performed, numerous environmental and technical variations can be created that can significantly influence the results obtained.

Moreover, the viewer device 1, 100 allows the evaluation of the dazzling recovery time to be carried out not only and not as has been done thus far, relating it to visual acuity but also to contrast sensitivity. All this is possible due to the digital technology never adopted in the past for these types of tests. After a dazzle, in fact, the contrast sensitivity is reduced in a very significant manner for a variable time depending on the subjects, during which the perception of images or figures is partially or totally canceled.

Examination of the chromatic sense

This test is preferably carried out through the presentation of 12 Ishihara tables. As known in the art, these are images consisting of small circles of different colors and constant brightness that the subject examined will be asked to recognize. They will all be clear to a subject with a normal chromatic sense, while they are difficult or impossible to perceive for those who have a relative or absolute lack with respect to a specific chromatic axis. Preferably, tables are presented relating to all the chromatic axes so as to highlight any type of discromatopsia. The viewer device 1, 100, through its processing unit, can be configured to analyze the responses and, through a reset program, define the type and degree of dichromatopsy possibly present.

In the present example, the result of this test is not related to the aforementioned Global Visual Index, but it can define the quality of the chromatic sense.

Hess screen

As known in the field, it is a test that allows the evaluation of ocular deviations related to paresis or paralysis of oculomotor muscles. It uses the diplopia induced by the lack of functional synergy of the pair of muscles that control the movements thereof in a certain gaze direction. This test allows graphically defining these deviations. Normally, this test is carried out by adopting a screen placed one meter away on which horizontal and vertical lines are drawn with a central fixation point and another eight peripheral points corresponding to the fields of action of the different pairs of synergistic muscles. In the conventional examination, red-green glasses are used, through which the patient is asked to superimpose a luminous green part of a torch that he is given on another red one that the examined subject will move to the different peripheral points corresponding to the fields of action of the different pairs of oculomotor muscles. For the examination of the contralateral eye, the examined subject and the examined subject will exchange the sights. In the presence of an ocular paresis, when the subject examined has the impression of superimposing the two traits actually diverges from them, due to the false projection of their images. In this way, two different diagrams are obtained depending on the fixing eye which describe the extent of the deviation in the different gaze positions.
In the case of the device 1, 100 the examined subject can move a luminous target in the points related to the fields of action of the different oculomotor muscle pairs following the localization on his screen, and the examined subject is asked to locate it. The different sight location points gathered by the examined subject will describe a graphical trace that allows highlighting and quantifying any deficits in the extrinsic ocular musculature.
Advantageously, during the whole test the examined subject can follow on the display of a computer connected to the device 1, 100, and possibly part of the processing unit mentioned above, the scenario presented to the examined subject in order to follow the execution of the test in every step thereof, giving the patient all the necessary indications for a correct execution in real time. At the end of the examination, a graph showing the possible deviation and its entity can be displayed to the examined subject.

As already mentioned, one or more of the aforementioned tests can be administered to the subject automatically, possibly under the guidance of a real or virtual operator, by means of the viewer device 1, 100. Moreover, the results can be acquired automatically, in particular through the aforementioned feedback means, and optionally displayed by the operator, in real time or not, by the processing unit associated with the device 1, 100 or by different means in communication with it. The results can also be stored in a memory unit associated with the processing unit.

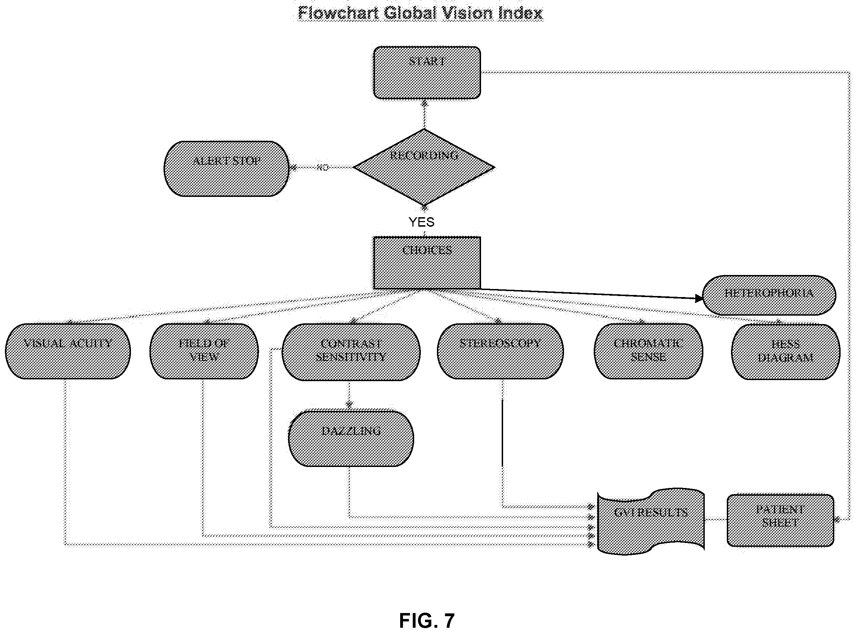

In a particularly preferred implementation, the method and the device of the invention allow the automatic calculation of the aforementioned Global Visual Index which, as mentioned, can also be defined as dynamic. It is suitable for quantifying the visual ability of a subject by combining different parameters, each representative of a specific capacity. The Global Visual Index can be calculated directly by the viewer device 1, 100, or by the apparatus that includes it, for example at the end of the tests described above. Figure 7 shows an exemplary flow diagram relating to a possible calculation method of said Index.

In a particularly preferred implementation, the method and the device of the invention can allow the automatic calculation of a particular visual index defined Advanced Visual Index adapted to the evaluation of the visual qualities of pilots participating in high-level motor competitions (for example motoring, motorcycling or motor-boating) or other particularly dynamic sports (e.g. bobsleigh, skydiving) or professional (airplane pilots) activities. For the calculation of this index we use the same algorithm used for the evaluation of the Global Visual Index as explained above, possibly modifying the parameters adopted for some eye examinations.
Some of the main variations that can be adopted are illustrated below.
The test for measuring visual acuity remains unchanged.

The visual field test will be carried out by reducing the brightness of the sights with respect to Table 3 for the calculation of the Global Visual Index according to the following scheme:

| Table 3 bis | | | | |
|---|---|---|---|---|
| Age range | Points up to 10 | Points 10 to 20 | Points 20 to 30 | Points 30 to 40 |
| 0-40 | 6% | 10% | 18% | 36% |
| 41-60 | 8% | 13% | 23% | 46% |
| Over 60 | 10% | 16% | 28% | 56% |

The evaluation of stereoscopy can instead be obtained through the values expressed in Table 4 through those expressed in the following Table 4bis.

| Table 4bis | |
|---|---|
| Quality level | Shift |
| Level 1 | 10" |
| Level 2 | 20" |
| Level 3 | 40" |
| Level 4 | 80" |
| Level 5 | 160" |

As far as the contrast sensitivity assessment is concerned, the test execution mode remains unchanged considering a dimension of the presented symbols equal to that of the fifth row of the optotype instead of the fourth, as foreseen by the procedure relative to the calculation of the Global Visual Index as introduced above.

In a particular meaning of the invention, in order to develop said specific Global Visual Index, in a first step dedicated to pilots and subsequently extended to all road vehicle drivers, the Inventors adopted a particular methodology, submitting different subjects to an eye examination for the measurement of various parameters, in particular those already indicated above.

The subjects then performed a simulator driving test, during which they were presented various scenarios specifically created, with the possibility of modulating various factors concerning vision to analyze them separately or in combination. The driving ability at the simulator was then quantified by one or more scores for each subject.

Through a methodology based on the application of fuzzy logic, combining the results of eye assessment with those obtained on the simulator test, each individual parameter was assigned a value, or weight, an expression of its relative importance. In this way, an algorithm was determined by which to calculate the aforementioned Index.

In particular, through the data collected in this way, a model of calculation was trained which can then be used in an inferential process of assessing the visual ability in target subjects.

An explanation of exemplary and preferred methods of calculating the Global Visual Index are given below.

A further method of calculating a Global Visual Index is described below, in particular via the viewer devices 1, 100 described above.

The base value (optimal reference IVG) can be equal to a predetermined higher threshold, in particular to a base value of 100, for a subject having the highest quality in each parameter examined. The base value is equal to that of binocular visual acuity. For example, a binocular visus 10/10 corresponds to a base value of 100 and a base value of 90 corresponds to a binocular visus of 9/10.

A first filter is applied, that is, a first test, relating to the residual field of view, in ways similar to those defined in the previous section "Visual field test" (also with reference to Figures 6 and 7). This residual field of view is defined by the ratio between the total value of the points perceived and the value of the total points taking into account their number and relative importance thereof which naturally decreases with the increase of their eccentricity in the following mode (in particular based on a total of 77 points distributed in four different areas):

- area included in the central 10° (8 points with a coefficient of relative importance 8),

- total value of points 64,

- area between 10° and 20° (20 points with a coefficient of relative importance 4)

In the previous example, the total value of the points is 80.

In the case of an area between 20° and 30° (22 points with coefficient of relative importance 2), the total value of the points is 44.

In the case of an area over 30° (27 points with coefficient of relative importance 1), the total value of the points is 27.

The total value of the points actually valued is obtained by multiplying their number by their relative importance and is 215.

For example, if a subject who has a visual acuity of 10/10 perceives points of the visual field for a total value of 180 out of a total of 215, the handicap relative to this factor will be 16.28%, so after this filter its Visual Index will be 83.72 (100-16.28).

For example, if a subject has a binocular visual acuity of 9/10 and a residual field of view equal to 70% of the total, his visual index after the visual field filter will be 63 (90x0.70).

A second filter, or text, can be related to Stereoscopy.

The result of the stereoscopy evaluation test can be expressed through a scale of values with five levels of decreasing quality, for example:

Level 1 (excellent) Handicap applied equal to 0

Level 2 (good) Handicap applied equal to -2%

Level 3 (fair) Handicap applied equal to -5%

Level 4 (sufficient) Handicap applied equal to -10%

Level 5 (insufficient) Handicap applied equal to -20%

For example, if we take the subject who after the filter linked to the visual field had an IVG of 63 and if the same has a stereoscopic level of 3 (handicap -5%), his IVG after this filter will be 59.85 (95% of 63).

A third filter, or test, can be related to contrast sensitivity.

The result of the contrast sensitivity evaluation test can be expressed through a scale of values with five levels of decreasing quality, in particular:

Level 1 (excellent) Handicap applied equal to 0

Level 2 (good) Handicap applied equal to - 0.2%

Level 3 (fair) Handicap applied equal to -0.4%

Level 4 (sufficient) Handicap applied equal to -0.8%

Level 5 (insufficient) Handicap applied equal to -1.2%

If again we take for example the subject who after the previous filters had an IVG of 59.85 and if at the contrast sensitivity test the same obtains a level 5 result (handicap -1.2%) the new value of his IVG will be 59.13.

A fourth filter, or test, may be the one related to the dazzling recovery time.

The result of the dazzling recovery time evaluation test is expressed through a scale of values with three different levels of decreasing quality:

Level 1 (excellent) Handicap applied equal to 0

Level 2 (sufficient) Handicap applied equal to -0.15%

Level 3 (insufficient) Handicap applied equal to -0.3%

If again we take for example the subject who after all the previous tests had an IVG of 59.13 and if at the dazzling recovery time test the same obtains a level 3 result (handicap -0.3%), his IVG will be 58.95.

Before performing the evaluation of binocular visual acuity, a monocular visual acuity evaluation test can be advantageously carried out. If the examined subject has in one of the two eyes a visual acuity equal to or < than 2/10, the IVG value calculated according to the above criteria will be decreased by 10%. For example, if a monocle or a subject suffering from severe amblyopia (eye visus worse <2/10) had at the end of the procedure as explained above, an IVG of 65, it will be automatically reduced by 10% and then will become 58.50.

A particularly advantageous specific implementation of the Global Visual Index calculation algorithm is shown below.

Based on the tests described above, the following variables are defined:

A1 - Monocular visual acuity.
EXAMINATION 0. Indicate the worst result between the 2 eyes (Value from 1 to 10 tenths)
A2 - Binocular visual acuity.
EXAMINATION 1 (Value from 1 to 10 tenths).
A3 - Residual field of view.
EXAMINATION 2. Points not seen in the area included in the central 10° (Value from 0 to 8)
A4 - Residual field of view.
EXAMINATION 2. Points not seen in the area between 10° and 20° (Value from 0 to 20)
A5 - Residual field of view.
EXAMINATION 2. Points not seen in the area between 20° and 30° (Value from 0 to 22)
A6 - Residual field of view.
EXAMINATION 2. Points not seen in the area above 30° (Value from 0 to 27)
A7 - Stereoscopy
EXAMINATION 3 (Value from 1 to 5)
A8 - Contrast sensitivity.
EXAMINATION 4 (Value from 1 to 5)
A9 - Dazzling recovery time.
EXAMINATION 5 (Value from 1 to 3)

The "EXAMINATIONS" cited are preferably performed according to the methods expressed in the previous sections.

The Global Visual Index (IVG) is then calculated according to the following algorithm, expressed in Excel® language.

$$IVG=(SE(A2=1;10;SE(A2=2;20;SE(A2=3;30;SE(A2=4;40;SE(A2=5;50;SE(A2=6$$

$$;60;SE(A2=7;70;SE(A2=8;80;SE(A2=9;90;100)))))))))/100*(100-$$

$$((A3*8+A4*4+A5*2+A6)*100/215))*(1-$$

$$(SE(A7=1;0;SE(A7=2;2;SE(A7=3;5;SE(A7=4;10;20)))))\%)*(1-$$

$$(SE(A8=1;0;SE(A8=2;0,2;SE(A8=3;0,4;SE(A8=4;0,8;1,2)))))\%)*(1-$$

$$(SE(A9=1;0;SE(A9=2;0.15;0.3)))\%)*(1-(SE(A1<=2;10;0))\%)$$

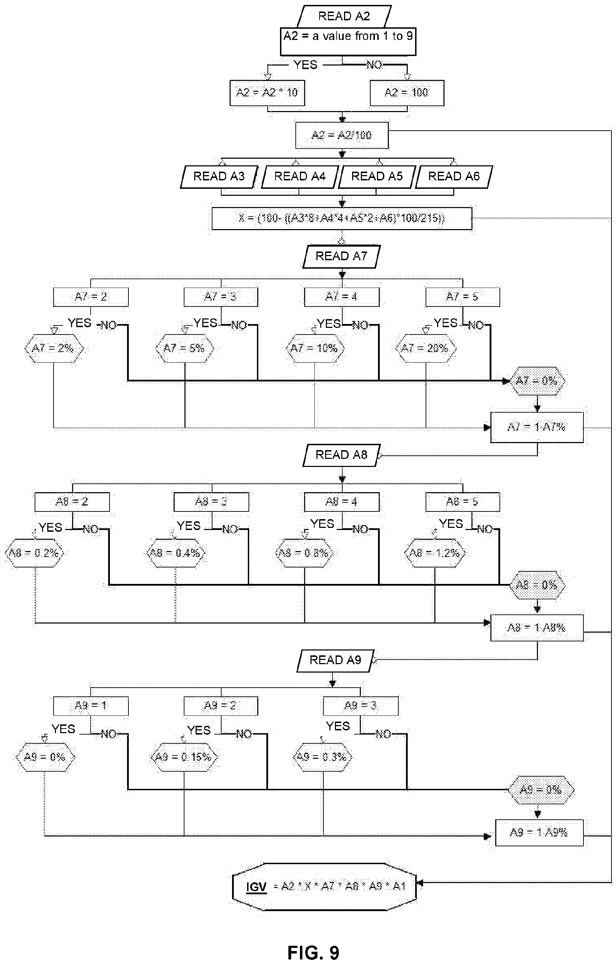

The same algorithm is shown in the flowchart in Figure 9.

A procedure adopted to define the relative importance coefficient of each individual visual factor in a specific implementation is described below.

Fifty patients undergoing eye examination were evaluated by evaluating the following visual parameters:

- Visual acuity
- Field of view
- Stereoscopy
- Contrast sensitivity
- Dazzling recovery time

A quantitative evaluation of the results relative to the examination of the individual parameters was carried out.
The same patients underwent a driving test with a highly immersive simulator.
Specific scenarios were created for this test, canceling the influence of the driving ability of the different drivers.

Within these scenarios, different sectors were created in which situations were distributed in which the different visual factors had a well calibrated weight and impact.

The difference was calculated between the value of the result achieved by each driver during the whole test and that of an optimal model, thus obtaining a global comparison.

The score obtained by the driver in the different sectors was evaluated and compared to that of an optimal model in the same sector, thus obtaining a comparative evaluation of the specific visual factor of that sector.

Through a methodology based on the application of fuzzy logic, combining the results of eye assessment with those obtained on the simulator test, each individual parameter was assigned a value, or weight, an expression of its relative importance. In this way, an algorithm was determined by which to calculate the IVG.

The data processing, carried out through the combination of the results obtained in the two test procedures conducted in a parallel and independent manner, allowed producing values expressing the relative importance of each single factor. This procedure was possible since in the fuzzy logic, the rules based on different inputs are independent of each other and do not influence the other units until the logical sum is executed. The individual rules are combined in order to constitute a structure within which it is possible to define a system that is however complicated in its entirety. But, due to the effects of parallel processing, it is the whole basis of rules that has a significant effect on the result. The defuzzification task aims to standardize all the results obtained from the elaboration of the rules and calculate the final value that must be made available to each output device.

Operations performed by a fuzzy logic system can be divided into two main groups. The inferential process consists of a list of rules and a logical sum operation: the rules can be divided into conditions (which form the antecedent block), and in conclusions (the resulting block). The conclusion is satisfied when all conditions are completely met.

It is important to remember that fuzzy reasoning, or the inferential process, starts with conditions for obtaining conclusions. Therefore, a logical sum and a defuzzification operation are needed to convert the results obtained from the inference process into a unique value.

The rules based on different inputs are independent of each other and do not influence the other units until the logical sum is executed. The individual rules are combined in order to constitute a structure within which it is possible to define a system that is however complicated in its entirety. But, due to the effects of parallel processing, it is the whole basis of rules that has a significant effect on the result. The defuzzification task aims to standardize all the results obtained from the elaboration of the rules and calculate the final value that must be made available to each output device.

Despite the simplicity of the expressions used, a very complex system can be developed in a rigorous manner. In this way, the synergy guaranteed by parallel processing allows fuzzy logic to proceed to the control of complex systems by resorting to particularly simple expressions. In addition, a fuzzy controller completes all processing steps without resorting to numerical calculations, thus increasing processing speed. Particularly high processing speeds can be obtained by using systems, implemented via hardware, that take full advantage of the potential offered by parallel processing. Therefore, it is possible to implement very complicated systems that can work in a precise, fast manner and guarantee high levels of reliability at the same time.

According to preferred methods of calculation of the aforesaid Index, the results of the tests relating to the evaluation of field of view, visual acuity, stereoscopy, contrast sensitivity and dazzling recovery time were selected as input variables, and such tests were preferably performed as illustrated above.

The output parameter is the aforementioned Global Visual Index.

To express the different conditions of the subject, up to seven codes were used, also called labels, for each parameter, as indicated in Table 7 below.

| Table 7 | |
| --- | --- |
| **Parameter: Field of view** | **Parameter: Visual acuity** |
| Very Insufficient (MI) | Very Insufficient (MI) |
| Insufficient (I) | Insufficient (I) |
| Almost Sufficient (AS) | Almost Sufficient (AS) |
| Sufficient (S) | Sufficient (S) |
| Almost Good (AG) | Almost Good (AG) |
| Good (G) | Good (G) |
| Excellent (E) | Excellent (E) |
| **Parameter: Stereoscopy** | **Parameter: Contrast sensitivity** |
| Insufficient (I) | Very Insufficient (MI) |

(continued)

| Parameter: Stereoscopy | Parameter: Contrast sensitivity |
|---|---|
| Sufficient (S) | Insufficient (I) |
| Good (G) | Sufficient (S) |
| Very Good (VG) | Good (G) |
| Excellent (E) | Really Good (RG) |
| **Parameter: Dazzling Recovery Time** | |
| Insufficient (I) | |
| Sufficient (S) | |
| Really Good (RG) | |

The Global Visual Index was evaluated according to the following Table 8.

| Table 8 | |
|---|---|
| Very Insufficient (MI) | Band 5 |
| Insufficient (I) | Band 4 |
| Sufficient (S) | Band 3 |
| Good (G) | Band 2 |
| Really Good (RG) | Band 1 |

In these modes, having adopted 7 membership functions each for the Visual Field and Visual Acuity variables, 5 for Stereoscopy and Contrast Sensitivity and 3 for Dazzling Recovery Time, one could have 3675 different combinations, using 5 output bands. The FAM (Fuzzy Associative Matrix) matrices were used, which show the input variables on the rows and columns, while inside the boxes there is the output variable.

As an example, below are exemplary FAM matrices for the different parameters.

STEREOSCOPY: Excellent (E)
FIELD OF VIEW

VISUAL ACUITY

| | MI | I | QS | S | QB | B | E |
|---|---|---|---|---|---|---|---|
| MI | 14 | 16 | 18 | 20 | 21 | 22 | 23 |
| I | 15 | 19 | 24 | 30 | 38 | 46 | 52 |
| QS | 16 | 22 | 34 | 50 | 54 | 58 | 64 |
| S | 17 | 25 | 40 | 60 | 63 | 67 | 72 |
| QB | 18 | 28 | 48 | 65 | 67 | 78 | 88 |
| B | 19 | 32 | 52 | 70 | 76 | 84 | 94 |
| E | 20 | 35 | 55 | 75 | 82 | 93 | 100 |

STEREOSCOPY: Very Good (VG)
FIELD OF VIEW

VISUAL ACUITY

| | MI | I | QS | S | QB | B | E |
|---|---|---|---|---|---|---|---|
| MI | 14 | 18 | 18 | 20 | 20 | 21 | 22 |
| I | 15 | 19 | 23 | 29 | 37 | 45 | 51 |
| QS | 16 | 21 | 33 | 49 | 53 | 57 | 62 |
| S | 17 | 24 | 39 | 59 | 61 | 65 | 70 |
| QB | 18 | 27 | 47 | 63 | 65 | 76 | 86 |
| B | 19 | 31 | 51 | 68 | 74 | 82 | 92 |
| E | 20 | 34 | 54 | 73 | 80 | 91 | 98 |

STEREOSCOPY: Good (G)
FIELD OF VIEW

VISUAL ACUITY

| | MI | I | QS | S | QB | B | E |
|---|---|---|---|---|---|---|---|
| MI | 13 | 15 | 17 | 19 | 20 | 21 | 22 |
| I | 14 | 18 | 23 | 29 | 36 | 44 | 49 |
| QS | 15 | 21 | 32 | 48 | 51 | 55 | 61 |
| S | 16 | 24 | 38 | 57 | 60 | 64 | 68 |
| QB | 17 | 27 | 46 | 62 | 64 | 74 | 84 |
| B | 18 | 30 | 49 | 67 | 72 | 80 | 89 |
| E | 19 | 33 | 52 | 71 | 78 | 88 | 95 |

STEREOSCOPY: Sufficient (S)
FIELD OF VIEW

VISUAL ACUITY

| | MI | I | QS | S | QB | B | E |
|---|---|---|---|---|---|---|---|
| MI | 13 | 14 | 16 | 18 | 19 | 20 | 21 |
| I | 14 | 17 | 22 | 27 | 34 | 41 | 47 |
| QS | 14 | 20 | 31 | 45 | 49 | 52 | 58 |
| S | 15 | 23 | 36 | 54 | 57 | 60 | 65 |
| QB | 16 | 25 | 43 | 59 | 60 | 70 | 79 |
| B | 17 | 29 | 47 | 63 | 68 | 76 | 85 |
| E | 18 | 32 | 50 | 68 | 74 | 84 | 90 |

STEREOSCOPY: Insufficient (I)
FIELD OF VIEW

VISUAL ACUITY

| | MI | I | QS | S | QB | B | E |
|---|---|---|---|---|---|---|---|
| MI | 11 | 13 | 14 | 16 | 17 | 18 | 18 |
| I | 12 | 15 | 19 | 24 | 30 | 37 | 42 |
| QS | 13 | 18 | 27 | 40 | 43 | 46 | 51 |
| S | 14 | 20 | 32 | 48 | 50 | 54 | 58 |
| QB | 14 | 22 | 38 | 52 | 54 | 62 | 70 |
| B | 15 | 26 | 42 | 56 | 61 | 67 | 75 |
| E | 16 | 28 | 44 | 60 | 66 | 74 | 80 |

The numerical value relative to the Global Visual Index is defined by the combination between the numerical value relative to the examination result of each single factor and its relative importance coefficient.
The Global Visual Index value can be entered by way of example, for example in the case of motor vehicle driving qualification within a predefined scale of values at 5 different levels in the manner of the following Table 9.

| Table 9 | |
|---|---|
| Level | Global Visual Index Value |
| Level 1 | 100-95 |
| Level 2 | 94-80 |
| Level 3 | 79-55 |
| Level 4 | 54-35 |
| Level 5 | < 35 |

The present invention has been described thus far with reference to preferred embodiments thereof. It is understood that other embodiments may exist that relate to the same inventive scope, as defined by the scope of protection of the following claims.

**Claims**

1. A digital viewer device (1), configured to allow a multifactorial visual ability test to be performed on a subject, the viewer device (1) comprising:

   - an outer casing (2) having a visual access window (3) for the subject's eyes;
   - first display means (4) housed within said casing (2), arranged in the visual field associated with said visual access window (3) and programmed or commanded to administer to the subject a first close view test;
   - second display means (5), programmed or commanded for administering to the subject a second view test from afar;
   - moving means (41, 42) of said first display means (4), configured to make said first display means (4) assume a first operative position, wherein the first display means (4) are in the visual field associated with said visual access window (3) for administering a first visual test to the subject, and a second rest position, wherein said first display means (4) are moved out of said visual field;

   wherein said second display means (5) are arranged spaced apart with respect to said visual access window (3), the overall configuration being such that said second display means (5) are operable to administer a second visual test to the subject when said first display means (4) are in said rest position.

2. The viewer device (100) according to claim 1, wherein said casing (2') has a substantially polyhedral conformation.

3. The viewer device (100) according to claim 1 or 2, wherein said first (4') and/or said second (5') display means comprise a programmed or programmable display (40', 50') for administering static and/or dynamic images, of a mono-ocular or bi-ocular type.

4. The viewer device (100) according to any one of the preceding claims, wherein said second display means (5') are arranged at an outer side wall (24', 25') of said casing (2').

5. The viewer device (100) according to any one of the preceding claims, wherein said second display means (5') comprise, or correspond to, a control panel operable by a user (102).

6. The viewer device (100) according to any one of the preceding claims, comprising a processing unit (10, 14, 15) configured to control said first (4') and/or said second (5') display means.

7. The viewer device (100) according to the preceding claim, wherein said processing unit (10, 14, 15) is housed within, or at, said casing (2').

8. The viewer device (100) according to any one of the preceding claims, comprising a processing unit (10, 14, 15) programmed for the calculation of a Global Visual Index from the results of the first and/or second visual test performed by said first (4') and/or said second (5') display means.

9. The viewer device (100) according to any one of the preceding claims, comprising a memory unit (110) configured to store results of one or more visual tests performed by said first (4') and/or said second (5') display means and preferably associated with said processing unit (10, 14, 15).

10. The viewer device (100) according to any one of the preceding claims, comprising a control panel (102) arranged at said casing (2') and communicating with said first (4') and/or said second (5') display means and/or with said processing unit (10, 14, 15).

11. The viewer device (100) according to any one of the preceding claims, comprising feedback means (111), operable by the subject during the visual tests.

12. The viewer device (100) according to any one of the preceding claims, configured to administer to the subject assessment tests of one or more of the following factors: natural and corrected monocular visual acuity of both eyes, natural and corrected binocular vision acuity, monocular and/or binocular visual field quality, binocular vision level and stereoscopic quality, contrast sensitivity, dazzling recovery time, any heterophoria degree, chromatic sense quality, ocular motility through the Hess screen.

13. The viewer device (1) according to any one of the preceding claims, wherein said moving means comprise a coupling between a pin (41) integral with said first display means (4) and a guide (42) obtained in said casing (2), preferably at a side wall (25) thereof.

**Patentansprüche**

1. Digitale Betrachtungsvorrichtung (1), die konfiguriert ist, um zu ermöglichen, einen multifaktoriellen Sehfähigkeitstest an einer Person auszuführen, wobei die Betrachtungsvorrichtung (1) umfasst:

   - ein äußeres Gehäuse (2) mit einem Sichtzugangsfenster (3) für die Augen der Person;
   - erste Anzeigemittel (4), die in dem Gehäuse (2) beherbergt sind, wobei diese in dem Gesichtsfeld angeordnet sind, das mit dem Sichtzugangsfenster (3) assoziiert ist, und die programmiert sind oder angewiesen werden, um die Person einem ersten Kurzsichttest zu unterziehen;
   - zweite Anzeigemittel (5), die programmiert sind oder angewiesen werden, um die Person einem zweiten Weitsichttest zu unterziehen;
   - Bewegungsmittel (41, 42) der ersten Anzeigemittel (4), die konfiguriert sind, um die ersten Anzeigemittel (4) in eine erste Betriebsposition zu bringen, wobei die ersten Anzeigemittel (4) sich in dem Sichtfeld befinden, das mit dem ersten Sichtzugangsfenster (3) assoziiert ist, um die Person einem ersten Sehtest zu unterziehen, sowie in eine zweite Ruheposition, wobei die ersten Anzeigemittel (4) aus dem Sichtfeld herausbewegt werden;

   wobei die zweiten Anzeigemittel (5) in Bezug auf das erste Sichtzugangsfenster (3) voneinander beabstandet angeordnet sind, wobei die Gesamtkonfiguration derart ist, dass die zweiten Anzeigemittel (5) betreibbar sind, um die Person einem zweiten Sehtest zu unterziehen, wenn sich die ersten Anzeigemittel (4) in der Ruheposition befinden.

2. Betrachtungsvorrichtung (100) nach Anspruch 1, wobei das Gehäuse (2') eine im Wesentlichen vielflächige Gestalt aufweist.

3. Betrachtungsvorrichtung (100) nach Anspruch 1 oder 2, wobei die ersten (4') und/oder die zweiten (5') Anzeigemittel eine programmierte oder programmierbare Anzeige (40', 50') umfassen, um statische und/oder dynamische Bilder eines monookularen oder biokularen Typs zuzuführen.

4. Betrachtungsvorrichtung (100) nach einem der vorhergehenden Ansprüche, wobei die zweiten Anzeigemittel (5') an einer äußeren Seitenwand (24', 25') des Gehäuses (2') angeordnet sind.

5. Betrachtungsvorrichtung (100) nach einem der vorhergehenden Ansprüche, wobei die zweiten Anzeigemittel (5') ein Steuerfeld umfassen oder einem solchen entsprechen, das von einem Nutzer (102) bedienbar ist.

6. Betrachtungsvorrichtung (100) nach einem der vorhergehenden Ansprüche, die eine Verarbeitungseinheit (10, 14, 15) umfasst, welche konfiguriert ist, um die ersten (4') und/oder die zweiten (5') Anzeigemittel zu steuern.

**7.** Betrachtungsvorrichtung (100) nach dem vorhergehenden Anspruch, wobei die Verarbeitungseinheit (10, 14, 15) in oder an dem Gehäuse (2') untergebracht ist.

**8.** Betrachtungsvorrichtung (100) nach einem der vorhergehenden Ansprüche, welche eine Verarbeitungseinheit (10, 14, 15) umfasst, die für die Berechnung eines globalen Seh-Indexes (Global Visual Index) aus den Ergebnissen des ersten und/oder zweiten Sehtests programmiert ist, die durch die ersten (4') und/oder die zweiten (5') Anzeigemittel ausgeführt werden.

**9.** Betrachtungsvorrichtung (100) nach einem der vorhergehenden Ansprüche, welche eine Speichereinheit (110) umfasst, die konfiguriert ist, um Ergebnisse von einem oder mehreren Sehtests zu speichern, die durch die ersten (4') und/oder die zweiten (5') Anzeigemittel ausgeführt werden, und die Vorzugsweise mit der Verarbeitungseinheit (10, 14, 15) assoziiert ist.

**10.** Betrachtungsvorrichtung (100) nach einem der vorhergehenden Ansprüche, welche ein Steuerfeld (102) umfasst, das in dem Gehäuse (2') angeordnet ist und mit den ersten (4') und/oder den zweiten (5') Anzeigemitteln und/oder mit der Verarbeitungseinheit (10, 14, 15) kommuniziert.

**11.** Betrachtungsvorrichtung (100) nach einem der vorhergehenden Ansprüche, welche Rückkoppelungsmittel (111) umfasst, die durch die Person während der Sehtests bedienbar sind.

**12.** Betrachtungsvorrichtung (100) nach einem der vorhergehenden Ansprüche, die konfiguriert ist, um die Person Einschätzungstests im Hinblick auf einen oder mehrere der nachfolgenden Faktoren zu unterziehen: Natürliche und korrigierte Monookulare Sehschärfe beider Augen, natürliche und korrigierte biokulare Sehschärfe, monookulare und/oder biokulare Sichtfeldqualität, biokulares Sehniveau und stereoskopische Qualität, Kontrastsensitivität, Blenderholungszeit, irgendein Heterophoriegrad, Farberfassungsqualität, okulare Motilität durch den Hess-Screen.

**13.** Betrachtungsvorrichtung (1) nach einem der vorhergehenden Ansprüche, wobei die Bewegungsmittel eine Kopplung zwischen einem Stift (41), der zu den ersten Anzeigemitteln (4) gehört und einer Führung (42), die in dem Gehäuse (2) vorliegt, umfasst, vorzugsweise an einer Seitenwand (25) davon.

## Revendications

**1.** Visionneuse numérique (1), configurée pour permettre à un test de capacité visuelle multifactoriel d'être réalisé sur un sujet, la visionneuse (1) comprenant :

- un logement externe (2) ayant une fenêtre d'accès visuel (3) pour les yeux du sujet ;
- un premier moyen d'affichage (4) contenu dans ledit logement (2), prévu dans le champ de vision associé à ladite fenêtre d'accès visuel (3) et programmé ou commandé pour administrer au sujet un premier test de vision de près ;
- un second moyen d'affichage (5), programmé ou commandé pour administrer au sujet un second test de vision de loin ;
- un moyen de déplacement (41, 42) dudit premier moyen d'affichage (4), configuré pour permettre audit premier moyen d'affichage (4) d'adopter une première position de fonctionnement, dans laquelle le premier moyen d'affichage (4) se trouve dans le champ de vision associé à ladite fenêtre d'accès visuel (3) afin d'administrer un premier test de vision au sujet, et une seconde position de repos, dans laquelle ledit premier moyen d'affichage (4) est déplacé en-dehors dudit champ de vision ;

dans laquelle ledit second moyen d'affichage (5) est prévu en étant espacé par rapport à ladite fenêtre d'accès visuel (3), la configuration globale étant telle que ledit second moyen d'affichage (5) est capable d'administrer un second test de vision au sujet lorsque ledit premier moyen d'affichage (4) se trouve dans ladite position de repos.

**2.** Visionneuse (100) selon la revendication 1, dans laquelle ledit logement (2') possède une conformation sensiblement polyédrique.

**3.** Visionneuse (100) selon la revendication 1 ou 2, dans laquelle ledit premier (4') et/ou ledit second (5') moyen d'affichage comprend un afficheur programmé ou programmable (40', 50') destiné à administrer des images statiques et/ou dynamiques, de type mono-oculaire ou bi-oculaire.

**4.** Visionneuse (100) selon l'une quelconque des revendications précédentes, dans laquelle ledit second moyen d'affichage (5') est prévu au niveau d'une paroi latérale externe (24', 25') dudit logement (2').

**5.** Visionneuse (100) selon l'une quelconque des revendications précédentes, dans laquelle ledit second moyen d'affichage (5') comprend, ou correspond à, un panneau de commande actionnable par un utilisateur (102).

**6.** Visionneuse (100) selon l'une quelconque des revendications précédentes, comprenant une unité de traitement (10, 14, 15) configurée pour commander ledit premier (4') et/ou ledit second (5') moyen d'affichage.

**7.** Visionneuse (100) selon la revendication précédente, dans laquelle ladite unité de traitement (10, 14, 15) est contenue dans, ou au niveau de, ledit logement (2').

**8.** Visionneuse (100) selon l'une quelconque des revendications précédentes, comprenant une unité de traitement (10, 14, 15) programmée pour le calcul d'un Indice Visuel Global à partir des résultats du premier et/ou du second test de vision réalisé par ledit premier (4') et/ou ledit second (5') moyen d'affichage.

**9.** Visionneuse (100) selon l'une quelconque des revendications précédentes, comprenant une unité de mémoire (110) configurée pour stocker les résultats d'un ou plusieurs tests de vision réalisés par ledit premier (4') et/ou ledit second (5') moyen d'affichage et de préférence associée à ladite unité de traitement (10, 14, 15).

**10.** Visionneuse (100) selon l'une quelconque des revendications précédentes, comprenant un panneau de commande (102) prévu au niveau dudit logement (2') et qui communique avec ledit premier (4') et/ou ledit second (5') moyen d'affichage et/ou avec ladite unité de traitement (10, 14, 15).

**11.** Visionneuse (100) selon l'une quelconque des revendications précédentes, comprenant un moyen de retour (111), actionnable par le sujet pendant les tests de vision.

**12.** Visionneuse (100) selon l'une quelconque des revendications précédentes, configurée pour administrer au sujet des tests d'évaluation d'un ou plusieurs des facteurs suivants : acuité visuelle monoculaire naturelle et corrigée des deux yeux, acuité visuelle binoculaire naturelle et corrigée, qualité du champ de vision monoculaire et/ou binoculaire, niveau de vision binoculaire et qualité stéréoscopique, sensibilité au contraste, temps de récupération après éblouissement, degré d'hétérophorie, qualité de détection chromatique, et mobilité oculaire à travers l'écran de Hess.

**13.** Visionneuse (1) selon l'une quelconque des revendications précédentes, dans laquelle ledit moyen de déplacement comprend un couplage entre une broche (41) qui fait partie intégrante dudit premier moyen d'affichage (4) et un guide (42) obtenu dans ledit logement (2), de préférence au niveau d'une paroi latérale (25) de celui-ci.

FIG. 1

FIG. 2

FIG. 3

FIG. 5

FIG. 4A

FIG. 4B

FIG. 6

Points presented with 1" interval;
Presentation time 1";
Randomized sequence
Background luminance: 10 dB

Age-related high-frequency
strategy (10 dB above threshold)
with repetition in unseen points

Within 10° = 8 points
Between 10° and 20° = 20
points
Between 20° and 30° = 22
points
Between 30° and 45° = 27
points
TOTAL = 77 points

Infinity focusing based on the refractive defect

MIRA IV
MIRA III

Flowchart Global Vision Index

FIG. 7

EP 3 629 883 B1

FIG. 8A

FIG. 8B

FIG. 8C

READ A2

A2 = a value from 1 to 9

YES — NO

A2 = A2 * 10

A2 = 100

A2 = A2/100

READ A3    READ A4    READ A5    READ A6

X = (100- ((A3*8+A4*4+A5*2+A6)*100/215))

READ A7

A7 = 2        A7 = 3        A7 = 4        A7 = 5

YES — NO    YES — NO    YES — NO    YES — NO

A7 = 2%      A7 = 5%      A7 = 10%      A7 = 20%

A7 = 0%

A7 = 1-A7%

READ A8

A8 = 2        A8 = 3        A8 = 4        A8 = 5

YES — NO    YES — NO    YES — NO    YES — NO

A8 = 0.2%    A8 = 0.4%    A8 = 0.8%    A8 = 1.2%

A8 = 0%

A8 = 1-A8%

READ A9

A9 = 1        A9 = 2        A9 = 3

YES — NO    YES — NO    YES — NO

A9 = 0%      A9 = 0.15%    A9 = 0.3%

A9 = 0%

A9 = 1-A9%

**IGV** = A2 * X * A7 * A8 * A9 * A1

**FIG. 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011027766 A
- US 2008189173 A